# EUROPEAN PATENT SPECIFICATION

(11) **EP 3 122 497 B1**
(45) Date of publication and mention of the grant of the patent: **17.06.2020**
(21) Application number: 15711547.8
(22) Date of filing: 24.03.2015
(51) Int. Cl.: B22F 3/11, A61L 27/06, A61L 27/56

(54) **METHOD FOR MANUFACTURING A POROUS METAL MATERIAL FOR BIOMEDICAL APPLICATIONS**
VERFAHREN ZUR HERSTELLUNG VON PORÖSEM METALLMATERIAL FÜR BIOMEDIZINISCHE ANWENDUNGEN
PROCÉDÉ DE FABRICATION D'UN MATÉRIAU MÉTALLIQUE POREUX POUR DES APPLICATIONS BIOMÉDICALES

(30) Priority: 24.03.2014 ES 201430408
(43) Date of publication of application: 01.02.2017
(73) Proprietor: ALEACIONES DE METALES SINTERIZADOS, S.A., 08980 Sant Feliu De Llobregat Barcelona (ES)
(72) Inventor: CALERO MARTÍNEZ, Jose, Antonio, E-08980 Sant Feliu De Llobregat (Barcelona) (ES); GIL MUR, Francisco, Javier, E-08980 Sant Feliu De Llobregat (Barcelona) (ES)
(74) Representative: Herrero & Asociados, S.L.
(86) International application number: PCT/EP2015/056253
(87) International publication number: WO 2015/144702

(56) References cited:
- WO-A1-03/013396
- WO-A1-2013/086504
- CN-A- 101 049 516
- US-A1- 2006 228 247
- POPA C ET AL: "Titanium-hydroxyapatite porous structures for endosseous applications", JOURNAL OF MATERIALS SCIENCE: MATERIALS IN MEDICINE, KLUWER ACADEMIC PUBLISHERS, BO, vol. 16, no. 12, 1 December 2005 (2005-12-01), pages 1165-1171, XP019212123, ISSN: 1573-4838, DOI: 10.1007/S10856-005-4724-5
- GLADIUS LEWIS: "Properties of open-cell porous metals and alloys for orthopaedic applications", JOURNAL OF MATERIALS SCIENCE: MATERIALS IN MEDICINE, vol. 24, no. 10, 13 October 2013 (2013-10-13), pages 2293-2325, XP055184842, ISSN: 0957-4530, DOI: 10.1007/s10856-013-4998-y
- UMHAUER H ET AL: "EFFECT OF PARTICLE SHAPE AND STRUCTURE ON THE RESULTS OF SINGLE- PARTICLE LIGHT-SCATTERING SIZE ANALYSIS", APPLIED OPTICS, OPTICAL SOCIETY OF AMERICA, WASHINGTON, DC; US, vol. 30, no. 33, 20 November 1991 (1991-11-20), pages 4980-4986, XP000241661, ISSN: 0003-6935, DOI: 10.1364/AO.30.004980
- RENLIANG XU ET AL: "Comparison of sizing small particles using different technologies", POWDER TECHNOLOGY - ELECTROSTATIC PHENOMENA IN PARTICULATE PROCESSES, ELSEVIER, BASEL (CH), vol. 132, no. 2-3, 24 June 2003 (2003-06-24), pages 145-153, XP002711749, ISSN: 0032-5910, DOI: 10.1016/S0032-5910(03)00048-2

## Description

### Field of the Art

The present invention relates to a porous metal material for certain biomedical applications. Particularly, the invention comprises a method for manufacturing and alloying titanium with improved osseointegration and adsorption properties.

### State of the Art

One of the major problems in selecting metal materials for biomedical arthroplasty applications is the need to combine two functional characteristics such as osseointegration and resemblance to human bone rigidity, all this under the initial premise of forming a material capable of withstanding dynamic loads in use.

Titanium has three major benefits: its great biocompatibility, reduced modulus of rigidity (110 GPa compared to 210 GPa of conventional sanitary steels) and compatibility with diagnostic and evaluation techniques such as CAT scans or MRIs. All this makes titanium or its alloys the most suitable metal materials for manufacturing any prosthesis or implant that must be placed inside the human body.

Forging material machined to the shape designed by specialists is used when forming the metal material for manufacturing these prostheses, the previously mentioned mechanical properties being maintained.

However, although these properties are better than those of any steel or CrCo material on the market, they are not enough to improve two basic aspects, i.e., bone resorption and osseointegration. The first of these aspects is closely linked to the difference between the modulus of rigidity of bone (0.5 at 30 GPa) and the modulus of rigidity of the metal prosthesis. As both values move closer to one another, the prosthesis acts functionally as a bone and resorption thereof is reduced, improving implant lifespan and therefore patient quality of life. The simplest way to reduce the modulus without modifying the material is by increasing system porosity, and the technology generating porous materials par excellence is powder metallurgy technology.

The second aspect of current material for prosthesis susceptible to improvement is osseointegration. Uncemented prostheses are increasingly used to reduce impact of the anchoring itself not only in the perforated cavity but in evaluating the risks of the set cement breaking. The use of uncemented prostheses involves development of anchoring systems themselves and the application of biocompatible coatings for ceramics or metal materials using thermal spraying technologies or the deposition of microspheres by small-scale gluing and resintering thereof is the most widely used today. Both types of systems have several drawbacks. Thermal spraying generates a rough surface, but there is no actual and in-depth intercommunication of this surface roughness, so the bone tissue only 'grips' the cavities and crests of the generated orography. In turn, the deposit of microspheres entails the inherent risk of detachment of some of these microspheres with the subsequent risk to patient health, given the small number of welding points said microspheres to be sintered have. Furthermore, in this latter case there have been various problems of the prosthesis fracturing while in service due to fatigue. The welded attachment of the sphere to the die generates sharp edges which are points with increased stresses.

Processing titanium material and alloys through powder metallurgy for obtaining porous material is known in the state of the art. The use of spacing agents that are removed in some of the processes of the production route to be followed is what has brought about the greatest success and seems to be what will be industrialized sooner.

The use of a spacer conditions the size of the pore generated in the part once the spacer is removed. However, the existence of the macropore does not necessarily entail the existence of channels having sizes similar to the macropore for blood capillary growth towards the inside of the porous material.

The powder metallurgy process allows compacting titanium and/or titanium alloy powders with a mean grain size from 300 micrometers to sizes less than 25 micrometers. This process followed by suitable sintering allows obtaining formed titanium materials with densities ranging between 85% and 98% of the solid due to the significant shrinkage observed during sintering. Given the fine grain structure, the mechanical properties are around the same values as the solid material when the parts are sintered under high vacuum conditions.

Stresses are transmitted to the bone through the prosthesis, so the prosthesis must have a modulus of elasticity as similar as possible to that of bone for suitable transfer of loads thereto and prevent the so-called mechanical stress shielding which causes bone resorption due to the lack of stress applied to the bone. Therefore, surface modulus of elasticity is of little importance if the prosthesis as a whole does not have a modulus similar to bone. If working with fine powder, load transmission during uniaxial compaction of metal powder generates density gradients involving distortions of the initial pressed shape during high-vacuum sintering. This is a significant drawback because it involves a process of machining to a final shape which is impossible to do after the sintering stage because the surface porosity closes and prevents osseointegration. The existence of distortions in sintering and particularly the existence of a 'skin' of laminated powder on the side surface of the part, due to friction existing during extraction from the mold, has led to the decision to perform green machining in order to open up porosity and seek the most suitable dimensions for attaining final shapes and measurements after sintering. Certain material strength is necessary for green machining, for which purpose some references and patents (for example US7674426B2) describe the use of cold isostatic compaction. All this makes the system more expensive and furthermore, since these processes are performed with fine titanium or titanium alloy powder, the final interconnection between said pores does not exceed 10 micrometers, so vascularization and efficient bone growth inside the porous system remains in question.

When a material is implanted in the body, an immune reaction to a foreign body occurs, causing the implant to be enveloped and encapsulated in fibrous tissue and isolated from surrounding tissue. This isolation is not of interest in certain applications because it would not allow attachment of the bone to the implant and the latter would not perform the functions for which it has been designed. To prevent the foregoing, it is necessary to convert the implant surface into a bioactive surface, i.e., capable of attaching to the adjacent bone tissue, or coating the surface with a material more similar to bone, such as hydroxyapatite. This hydroxyapatite usually has an amorphous character or low crystallinity, which entails very rapid rates of dissolution in blood medium, generating problems of prosthesis instability which end in operations for re-placing same as well as less or nil osseointegration.

WO 2013/086504 A1 discloses a method for fabricating porous metal constructs.

WO 03013396 A1 discloses a surgical implant comprising a core region and a porous surface region extending over at least part of said core region.

CN101049516 A discloses an imbedded body in porous titanium of biologic medical use, and a preparation method.

Other prior art is: POPA C ET AL, "Titanium-hydroxyapatite porous structures for endosseous applications", JOURNAL OF MATERIALS SCIENCE: MATERIALS IN MEDICINE, KLUWER ACADEMIC PUBLISHERS, BO, (20051201), vol. 16, no. 12, doi:10.1007/S10856-005-4724-5, ISSN 1573-4838, pages 1165 - 1171, XP019212123, page 1166 - page 1167, and GLADIUS LEWIS, "Properties of open-cell porous metals and alloys for orthopaedic applications", JOURNAL OF MATERIALS SCIENCE: MATERIALS IN MEDICINE, (20131013), vol. 24, no. 10, doi:10.1007/s10856-013-4998-y, ISSN 0957-4530, pages 2293 - 2325, XP055184842 page 2293 - page 2296 and page 2299 - page 2300.

### Object of the Invention

To solve the mentioned problems, the method of the invention proposes the use of a titanium powder with specific properties and the mixture thereof with a salt of a specific size and also at a specific proportion. More specifically, the invention proposes a method for obtaining a porous titanium part according to claim 1.

These features result in an interconnection between pores of more than 150 micrometers, and together with the biocompatible nature of the titanium-based material, they make the product very suitable for improving osseointegration of the material while maintaining suitable fatigue strength.

### Brief Description of the Drawings

For the purpose of aiding to better understand the features of the invention according to a preferred practical embodiment thereof, a set of drawings is attached to the following description, in which drawings the following has been depicted with an illustrative character:
Figure 1a is an electron microscopy image of the irregular titanium powder used for the invention with a mean size of 200 micrometers.
Figure 1b is an image of a microstructure of a part produced according to the invention where the pore size and interconnection of more than 150 micrometers between pores can be seen.
Figure 2a is a graph showing the fracture compression behavior of a part produced according to the method of the invention.
Figure 2b is a graph showing the fatigue behavior under conditions of service of an intersomatic cage made according to the method of the invention.
Figure 3 depicts the size of the porosity with respect to the logarithm of the mean volume that can be occupied by a material (intrusion) for a structure manufactured according to the claimed method.
Figure 4 is a photograph showing the high crystallinity of a part produced according to the invention.
Figure 5 is a photograph of the morphology of osteoblastic cells on the surface of the porous material.

### Detailed Description of the Invention

A general aspect of the invention starts from pure titanium powder with a particle size distribution between 45 and 300 micrometers, with more than 90% of the particles between 75 and 250 micrometers and a mean size of 200 micrometers. The flow rate of said powder is 93s, its apparent density is 1.0 g/cm³ and its tap density is 1.25 g/cm³.

Flow rate of the material is calculated according to ISO 4490 standard, apparent density is calculated according to ISO 3923/1 and tap density is calculated according to ISO 3953. An apparent density of 22% of the solid material density (1.0 g/cm³ with respect to solid titanium density of 4.51 g/cm³) indicates the existence of a highly irregular powder surface which, together with the mean particle size of 200 micrometers and salt size of 300 micrometers to 600 micrometers, makes attaining interconnection sizes between pores greater than 150 micrometers using a pressing and sintering process feasible.

Titanium at a proportion of 34% by weight is mixed with NaCl between 300 and 600 micrometers and 50% and 80% by weight, a binder being added at a proportion of at least 15% to 100%. Subsequently, in order to remove the binder and salt, the material is subjected to a thermal process followed by continuous rinsing in double-distilled water and after compacting the material between 200 and 400 MPa, preferably 300 MPa, it is sintered at a temperature between 1200°C and 1400°C (preferably 1300°C) and at a pressure less than 4· 10⁻⁴ mbar. Suitable porosity and length between pores in addition to suitable strength and homogeneity are achieved with these parameters.

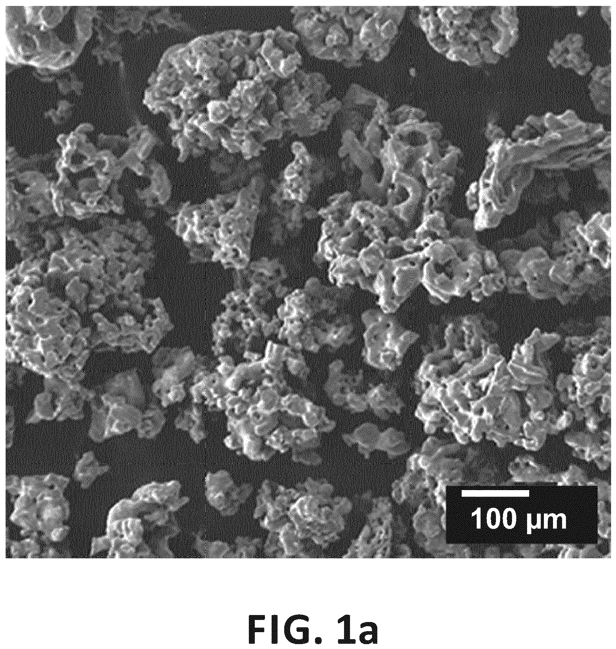

As seen in Figure 1a, the titanium powder has an irregular morphometry and size greater than 150 micrometers.

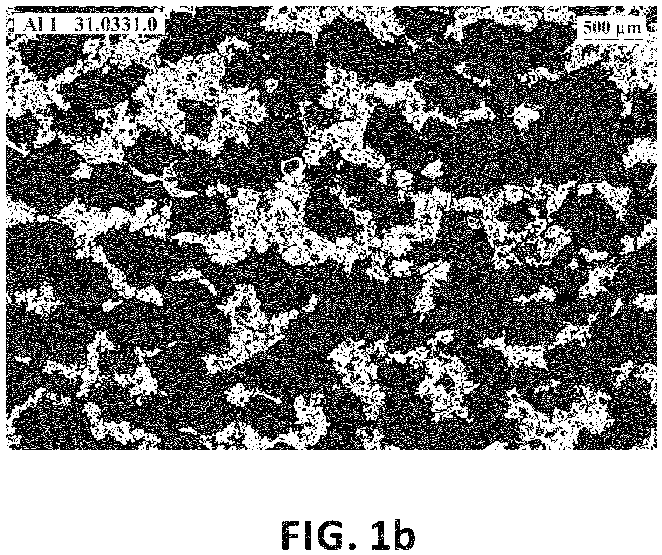

As can be seen in Figure 1b, the structure of the developed material is completely interconnected with interconnection sizes greater than 150 micrometers.

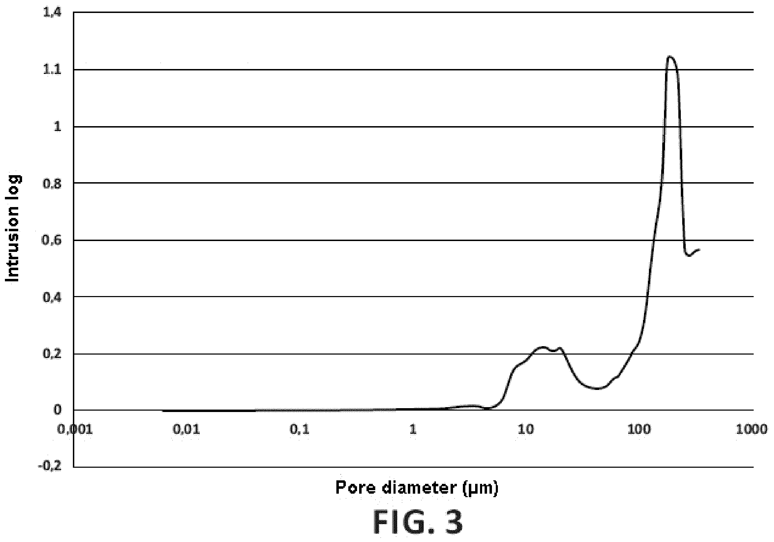

Figure 3 shows the plotted result of mercury porosimetry. In a porous intercommunicated structure like in this case, the most recurrent maximum intensity value is the size of the intercommunication channel between pores. Therefore, the most intense second peak represents the size of the interconnected porosity which is greater than 150 micrometers. The first peak represents all internal porosities of less than 10 micrometers which are inside the material and will have a specific biological function during the osseointegration process, acting as 'food' storage for said cell growth.

In order to convert the titanium surface into a bioactive surface, the passive titanium oxide (TiO₂) layer, which is produced spontaneously in titanium and its alloys, of the implant surface is reacted with a 5 M basic sodium hydroxide (NaOH) solution. TiO₂ partially dissolves during treatment with NaOH to form an alkaline solution as a result of the corrosive attack of the hydroxyl groups (OH⁻) of the solution. As a result, a sodium titanate (Na₂TiO₃) gel layer is formed on the surface. The basic reaction is then neutralized by means of H₂O at a temperature of 60°C for 24 hours. A heat treatment is subsequently performed at 600°C for 1 hour to dehydrate, densify and increase substrate adhesion of this sodium titanate gel layer. A stable and partially crystalline Na₂TiO₃ layer which promotes bioactivity and improves surface properties is thus formed.

### Example. Intersomatic cage:

Pure titanium grade 2 powder with a particle size distribution between 45 and 300 micrometers and a mean particle size of 200 micrometers was used. 65% by volume of NaCl with a size comprised between 300-600 micrometers was introduced. 15% ethylene glycol was added to the final mixture. It was mixed in a double cone blender for 10 minutes. The wet mixture was introduced in the die with the final geometry. The die was oversized by 8% due to the homogeneous shrinkage of the material during sintering. Uniaxial pressing is performed at 300 MPa in a hydraulic press, and the excess binder acts to facilitate homogenous distribution of pressure during ejection from the die. Due to this effect, the transmission of compaction pressure is very homogenous, so the green density of the compact material is also homogenous and does not cause distortions due to shrinkage difference in the final sintered part. The pressed parts with titanium and salt are passed through an oven at 200°C for 6 hours to remove ethylene glycol residues (the ethylene glycol evaporation temperature is close to 190°C). At 200°C, there is no risk of oxygen being incorporated in the titanium structure, so possible contaminations with said element are minimized. A cyclic process of baths is subsequently performed to remove the spacer until ionic conductivity stabilizes at values that are very small or similar to those of the distilled water used as a solvent. Said washing is performed by applying a vacuum to accelerate the salt dissolution process. Once salt has been removed from the part, it is properly handled and left for 4 hours at 120°C in an air oven to dry it completely.

It is subsequently sintered in a high-vacuum furnace (<4·10⁻⁴ mbar) at 1200°C-1400°C, preferably 1300°C, for 4 hours. Once sintered, the samples are machined, rounding off the edges and reducing the possibility of particle detachment. Finally, cyclic processes of washing in distilled water, alcohol and acetone are performed, all under ultrasound, to properly clean the parts.

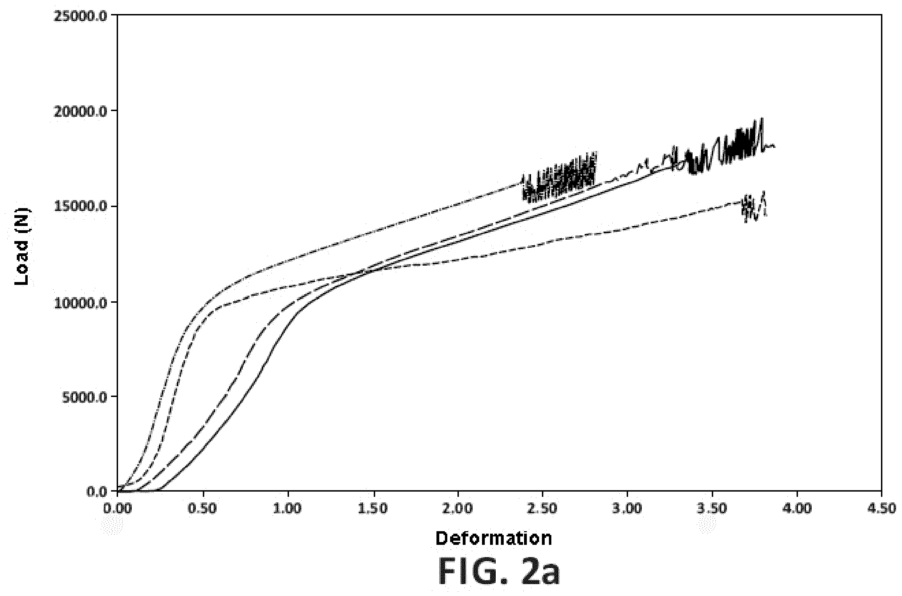

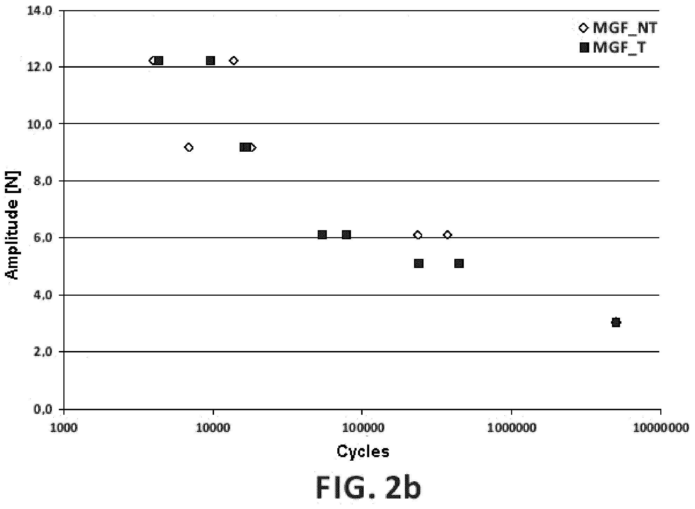

Compression tests were conducted on treated and untreated samples, the results of which did not allow observing significant differences in term of elastic limit, fracture load or fracture elongation. Nevertheless, a certain tendency towards an increase in the modulus of elasticity of the material when performing bioactivity thermal treatment was observed. The mechanical compression strength and modulus of elasticity (10 GPa) values, as well as the good fatigue behavior thereof, allow assuring good mechanical behavior of this type of porous material for intersomatic cage applications for the spinal column and opens up the possibility to many other applications for hard tissue replacement. Figures 2a and 2b show good compression behavior with monotonic bending and fatigue, simulating how the spinal column works. In these mechanical tests, infinite life values exceed 350 kg during the service life and there is no particle detachment whatsoever.

In order to evaluate the capacity of the implanted material to form an apatite layer on the surface thereof, an in vitro test was performed with a sample manufactured according to the method of the invention, following the guidelines of the international ISO 23317 standard (Implants for surgery - In vitro evaluation for apatite - forming ability of implant materials). This test consists of submerging the material in a solution with ion concentration, pH and temperature nearly equal to blood plasma, which is referred to as simulated body fluid or SBF.

Bioactivity evaluation by means of SBF is evaluated according to apatite formation on the surface due to ion exchange generated between SBF and the chemically and thermally treated surface.

When thermally-chemically treated titanium is immersed in SBF, Na⁺ ions of Na₂TiO₃ are exchanged with H₃O⁺ ions of the aqueous medium and Ti-OH groups are formed on the metal surface. The Ti-OH groups that are formed on the surface are combined with calcium Ca²⁺ ions of the SBF to form amorphous calcium titanate (CaTiO₃). The Ti-OH groups on the Ti surface are electrically charged and cause Ca²⁺ ions to precipitate on the surface in order to combine with them.

Part of the calcium ions reacts with HPO₄²⁻ phosphates of SBF to form calcium phosphate (CaP) on the implant surface. The release of sodium Na ions ⁺, along with H₃O⁺ ions of SBF, results in an increase in solution pH. This in turn produces greater CaP ion activity causing rapid deposition of apatite on the titanium surface.

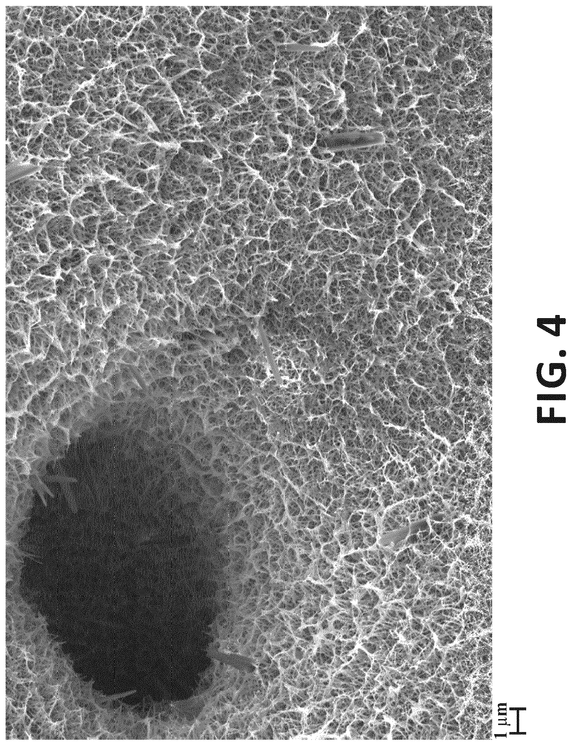

After thermal-chemical treatment, the samples offer a microstructure such as that observed in Figure 4. It can be seen that sodium titanate covers the entire surface and even the porosity because treatment penetrates the entire implant surface since it is a liquid treatment. High crystallinity can be seen and attachment with the implant is assured because it is not a coating but a titanium-based crystallization process. This assures inhibition of possible bacterial filtration, as occurred in implants with bioactive coatings, because there were a few micrometers between the layer and the substrate that bacteria and microorganisms used to form colonies.

Samples treated with different amounts of basic solution and subsequently by means of thermal treatment were immersed in SBF for 10 days to determine surface bioactivity, as indicated in the ISO 23317 standard. The crystalline structure of the formed apatite could be confirmed upon immersion. It is very important for the apatite to be crystalline. In implants coated with hydroxyapatite by plasma, apatite was for the most part rendered amorphous. This produced very rapid dissolution of the apatite layer with the physiological medium, and the implant and bone remained separated by a distance such that osseointegration did not occur. In this case, the apatite is completely crystalline and its dissolution is much slower than amorphous apatite, which is optimal for osseointegration processes.

Based on the absorbance values obtained for SAOS-2 osteoblasts after 24 hours of incubation and 10 minutes of development with LDH reagent, it can be asserted that:
- Relevant cytotoxic effects due to indirect exposure of SAOS-2 cells to 5 concentrations of extracts obtained from the analyzed samples have not been observed.
- The absorbance value with respect to the negative control is above 75% in all the samples, so it is within the values allowed in which cytotoxicity can be considered non-existent.

An increase in proliferation between days 1 and 14 was observed because the cells had not started to differentiate and proliferate. A certain decrease in proliferation was subsequently observed due with almost absolute certainly to the increase in cell differentiation after 14 days of incubation. All this behavior is normal in such cells. ALP activity also increased after 7 days, indicating cell differentiation. ALP is an indicator of the start of differentiation, and a drop in phosphatase activity after 14 days of culture, after an increase in activity after 7 days of incubation, is considered normal. This phenomenon is highly categorized in scientific literature as typical early cell differentiation process. As the culture time increases, the number of cells and the degree of penetration thereof into the material also increases. Images of the samples incubated for 14 days already show a degree of complete penetration into the sample, which would amount to half the thickness of the component.

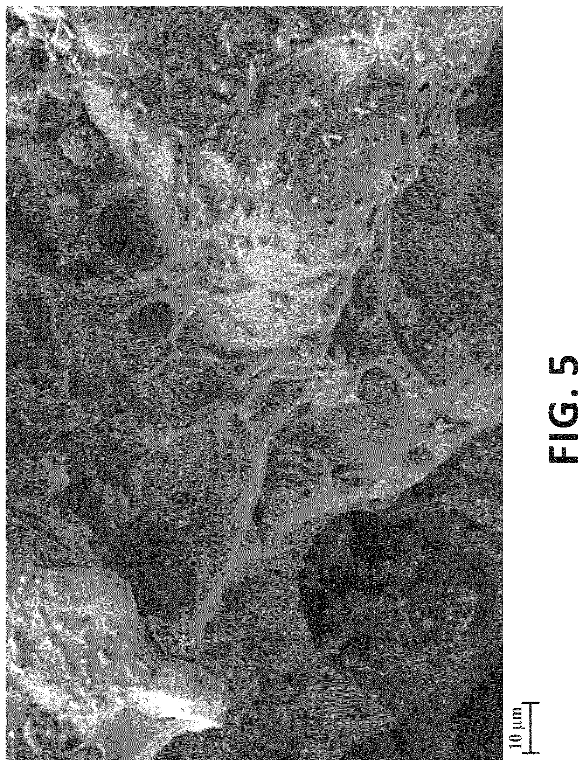

Figure 5 shows the morphology of osteoblastic cells on the surface of the porous material from AMES with a very high degree of focal points, which assures good cell adhesion and health, as shown by the osteocalcin levels found. The adhesion, proliferation and differentiation contrasted with high osteocalcin and gene expression levels assure the acceleration of osseointegration.

## Claims

1. Method for obtaining a porous titanium part, **characterized in that** the starting titanium powder is pure grade 2, with a mean particle size of 200 micrometers, a flow rate of 93s calculated according to ISO 4490 standard, an apparent density of 1.0 g/cm³ calculated according to ISO 3923/1, and said powder is mixed at a proportion of 34% titanium by weight with NaCl with a particle size between 300 and 600 micrometers and at least 50% by weight of NaCl, and further **characterised in that** it comprises the following steps:
i) adding a binder at a proportion of at least 15%;
ii) compacting the resulting material between 200-400 MPa;
iii) removing the binder and salt by means of a thermal process followed by continuous rinsing in double-distilled water;
iv) sintering at a temperature between 1200°C and 1400°C and at a pressure less than 4.10⁻⁴ mbar;
v) machining the surface of the part.

2. Method according to claim 1, **characterized in that** the part is introduced in a 5 M basic sodium hydroxide (NaOH) solution after step v).

3. Method according to any of claims 2 **characterized in that** the binder is ethylene glycol.

4. Method according to any of claims 2-3, **characterized in that** step i) is performed in a double cone blender and step iii) is performed by means introducing the wet mixture in a die and subsequent uniaxial pressing.

5. Method according to claim 4, **characterized in that** uniaxial pressing is performed in a hydraulic press.

## Patentansprüche

1. Verfahren zum Erhalten eines porösen Titanteils, **dadurch gekennzeichnet, dass** das Ausgangstitanpulver Reinheitsgrad 2, mit einer mittleren Teilchengröße von 200 Mikrometer, einer Fließgeschwindigkeit von 93 Sekunden berechnet gemäß ISO Standard 4490, einer scheinbaren Dichte von 1,0 g/cm³ berechnet gemäß ISO 3923/1 ist und das Pulver gemischt ist bei einem Anteil von 34 Gew.-% Titan mit NaCl mit einer Teilchengröße zwischen 300 und 600 Mikrometer und zumindest 50 Gew.-% an NaCl, und ferner **dadurch gekennzeichnet, dass** es die folgenden Schritte umfasst:
i) Zugabe eines Bindemittels bei einem Anteil von zumindest 15%;
ii) Verdichten des resultierenden Materials zwischen 200-400 MPa;
iii) Entfernen des Bindemittels und Salzes durch einen thermischen Prozess gefolgt von kontinuierlichem Spülen in doppelt destilliertem Wasser;
iv) Sintern bei einer Temperatur zwischen 1200°C und 1400°C und bei einem Druck von weniger als 4,10⁻⁴ mbar;
v) Bearbeiten der Oberfläche des Teils.

2. Verfahren nach Anspruch 1, **dadurch gekennzeichnet, dass** der Teil nach Schritt v) in eine 5 M basische Natriumhydroxid (NaOH) -Lösung eingeführt wird.

3. Verfahren nach Anspruch 2, **dadurch gekennzeichnet, dass** das Bindemittel Ethylenglycol ist.

4. Verfahren nach einem der Ansprüche 2-3, **dadurch gekennzeichnet, dass** Schritt i) in einem Doppelkegelmischer durchgeführt wird und Schritt iii) durch Einbringen der feuchten Mischung in eine Düse und anschließendes einachsiges Pressen durchgeführt wird.

5. Verfahren nach Anspruch 4, **dadurch gekennzeichnet, dass** ein einachsiges Pressen in einer hydraulischen Presse durchgeführt wird.

## Revendications

1. Procédé pour obtenir une pièce en titane poreuse, **caractérisé en ce que** la poudre de titane de départ est de pureté grade 2, avec une granulométrie moyenne de 200 micromètres, un indice d'écoulement de 93s calculé conformément à la norme ISO 4490, une masse volumique apparente de 1,0 g/cm³ calculée conformément à la norme ISO 3923/1, et ladite poudre est mélangée en une proportion de 34 % en poids de titane avec du NaCI ayant une granulométrie comprise entre 300 et 600 micromètres et au moins 50 % en poids de NaCI, et **caractérisé en outre en ce qu'**il comprend les étapes suivantes :
i) addition d'un liant en une proportion d'au moins 15 % ;
ii) compactage du matériau résultant entre 200 et 400 MPa ;
iii) retrait du liant et du sel au moyen d'un traitement thermique, suivi d'un rinçage continu dans de l'eau doublement distillée ;
iv) frittage à une température comprise entre 1200 °C et 1400 °C et sous une pression inférieure à 4 x 10⁻⁴ mbar ;
v) usinage de la surface de la pièce.

2. Procédé selon la revendication 1,
**caractérisé en ce que** la pièce est introduite dans une solution basique d'hydroxyde de sodium (NaOH) 5 M après l'étape v).

3. Procédé selon la revendication 2,
**caractérisé en ce que** le liant est l'éthylèneglycol.

4. Procédé selon l'une quelconque des revendications 2 à 3,
**caractérisé en ce que** l'étape i) est réalisée dans un mélangeur à double cône et l'étape iii) est réalisée au moyen de l'introduction du mélange humide dans une matrice et d'un pressage uniaxial subséquent.

5. Procédé selon la revendication 4,
**caractérisé en ce que** le pressage uniaxial est réalisé dans une presse hydraulique.
